# EUROPÄISCHE PATENTSCHRIFT

(11) **EP 1 150 702 B1**
(45) Veröffentlichungstag und Bekanntmachung des Hinweises auf die Patenterteilung: **23.10.2002**
(21) Anmeldenummer: 00903429.9
(22) Anmeldetag: 15.02.2000
(51) Int. Cl.: A61K 38/17, A61K 47/42, A61P 1/18

(54) **VERWENDUNG VON OROSOMUCOID ZUR HERSTELLUNG EINER PHARMAZEUTISCHEN PRÄPARATION ZUR BEHANDLUNG VON PANKREATITIS**
USE OF OROSOMUCOID FOR THE PRODUCTION OF A PHARMACEUTICAL PREPARATION FOR THE TREATMENT OF PANCREATITIS
UTILISATION D'OROSOMUCOIDE POUR LA PREPARATION D'UNE COMPOSITION PHARMACEUTIQUE DESTINEE AU TRAITEMENT DE LA PANCREATITE

(30) Priorität: 18.02.1999 AT 27199
(43) Veröffentlichungstag der Anmeldung: 07.11.2001
(73) Patentinhaber: Baxter Aktiengesellschaft, 1220 Wien (AT)
(72) Erfinder: AUER, Wilfried, A-1220 Wien (AT); MUCHITSCH, Eva-Maria, A-1140 Wien (AT); PICHLER, Ludwig, A-1040 Wien (AT); SCHWARZ, Hans-Peter, A-1180 Wien (AT)
(74) Vertreter: Pawloy, Heinrich, Dr.
(86) Internationale Anmeldenummer: AT0000038
(87) Internationale Veröffentlichungsnummer: WO00048624

(56) Entgegenhaltungen:
- WO-A-98/12225
- WO-A-98/40087
- WILLIAMS JP ET AL: "Alpha1-acid glycoprotein reduces local and remote injuries after intestinal ischemia in the rat" AMERICAN JOURNAL OF PHYSIOLOGY: GASTROINTESTINAL AND LIVER PHYSIOLOGY, Bd. 36, Nr. 5, November 1997 (1997-11), Seiten g1031-G1035, XP000939066 in der Anmeldung erwähnt

## Beschreibung

Die Erfindung betrifft eine neue Verwendung von Orosomucoid.

α₁-saures Glycoprotein (AGP), welches auch Orosomucoid genannt wird, ist eine aus Plasma gewonnene Substanz, welche ein Molekulargewicht von 40 000 Dalton aufweist, und einen Kohlehydratanteil zwischen 30 und 50 % aufweist. AGP besteht aus einer einzigen Polypeptidkette mit 183 Aminosäuren und weist zwei Disulfidbrücken auf. Es umfasst weiters fünf Kohlehydratketten, welche alle in der ersten Hälfte der Peptidkette lokalisiert sind. Diese Kohlehydratgruppen bestehen zu rund 14 % aus neutralen Hexosen, 14 % Hexosaminen, 11 % Sialinsäure und 1 % Fructose. Je nach Quelle der AGP-Präparation bzw. Gewinnungs- oder Charakterisierungsmethode tritt AGP in unterschiedlichen Formen auf, welches auf Unterschiede in den Kohlehydratketten zurückgeführt wird.

Die Eigenschaften und biologischen Funktionen von Orosomucoid sind in den Übersichtsartikeln von Schmid (in "The Plasma Proteins Structure Function and Genetic control", Vol. 1 (1975), Academic Press, Ed. Frank A. Putnam, 2^{nd} Edition, Seiten 183-228) und Kremer et al. (Pharmacological Reviews 40 (1988), Seiten 1-47), beschrieben.

In der Medizin wurde AGP bislang als wesentliche Trägersubstanz für vorwiegend basische Medikamente in Plasma erachtet (siehe Kremer et al.).

Weiters konnte eine positive Wirkung von Orosomucoid bei Entzündungsreaktionen nachgewiesen werden. So beschrieben Denko et al. (Agents and Actions 15, 5/6 (1984), 539-540) eine antiinflammatorische Wirkung von AGP bei Uratkristallentzündungen in Ratten. Libert et al. (J. Exp. Med. 180 (1994), 1571-1575) wiesen nach, dass eine ähnliche Indikation in der Verhinderung von septischem Schock im Zusammenhang mit der Wirkung von TNF-α oder Lipopolysacchariden liegt.

Verbunden mit dieser antiinflammatorischen Wirkung von Orosomucoid konnte gezeigt werden, dass auch lokale Schäden und "remote"-Schäden, wie "remote lung injury", nach intestinaler Ischämie in der Ratte mit Orosomucoid behandelt werden können (Williams et al., Am. J. Physiol. 273 (1997), G1031-1035).

Die Verwendung von Orosomucoid zur Behandlung von Störungen der Durchblutung und der Mikrozirkulation nicht-inflammatorischer Art, insbesondere zur Behandlung von hämorrhagischem und/oder hypovolämischem Schock, ist in der WO 98/40087 beschrieben.

Prinzipiell wird zwischen akuter und chronischer Pankreatitis unterschieden, wobei die Ursachen sowohl von akuter als auch von chronischer Pankreatitis sehr verschieden sein können; in rund 20 % der Fälle bleibt die Ursache überhaupt unbekannt.

Mit Pankreatitis wird eine Entzündung des Pankreas bezeichnet, die mit einer Autolyse dieses Organs verbunden ist, die pathologisch/anatomisch, je nach Schweregrad mit interstitiell ödematösen Veränderungen bis zu hämorrhagischer Pankreasnekrose verbunden ist.

Akute Pankreatitis tritt gehäuft bei Alkoholkrankheit, Gallenwegserkrankungen, Papillenstenose, postoperativ nach chirurgischen Eingriffen in der Bauchhöhle, Hyperlipoproteinämien, Hyperparathyreoidismus, Ulcus ventriculi, Mumps und nach Einnahme von Glukokortikoiden auf. Auch immunologische Ursachen wurden postuliert, es konnte aber bislang noch kein überzeugender Beleg gefunden werden, dass immunologische Faktoren für die Entstehung von akuter oder chronischer Pankreatitis beim Menschen verantwortlich gemacht werden könnten.

Die Letalität variiert je nach Schweregrad der Pankreatitis zwischen 15 und 20 %, wobei sie bei schweren Formen bis zu 80 % beträgt. Beger et al. (World J. Surg. 21 (1997), S. 130-135) identifizieren ödematöse Pankreatitis und nekrotisierende Pankreatitis als die häufigsten, klinischen Manifestationen. Der mit akuter Pankreatitis zusammenhängende, multiple Organschaden wird auf verschiedene Entzündungsmediatoren zurückgeführt, welche auf die Nekroseprozesse im Rahmen der Pankreatitis zurückzuführen sind. Es kann auch zu lokalen und systemischen septischen Komplikationen kommen. Im Allgemeinen wird davon ausgegangen, dass diese nekrotisierenden Prozesse der Pankreatitis durch eine vorzeitige Aktivierung der Pankreasenzyme verursacht werden. Für eine derartige frühzeitige Aktivierung müssen jedoch eine ganze Anzahl von Schutzmechanismen in den azinaren Zellen des Pankreas überwunden werden. Erst in weiterer Hinsicht sind dann Prozesse wie Gallensteine oder Störungen des Sphincter-Oddi oder der duodenalen Papille, welche ein Eindringen von Gallenflüssigkeit oder von Flüssigkeit des Zwölffingerdarms in den Ductus pankreaticus verursachen, wesentlich (Lerch et al., Int. J. Pancreatol. 15 (3) (1994), S. 159 - 170).

Bedingt durch die vielen möglichen Ursachen, die eine Pankreatitis haben kann, wurden auch zahlreiche invasive und chemische Therapien vorgeschlagen, welche teilweise auch kombiniert angewendet werden. Zu diesen Therapien gehören die exkretorische Ruhigstellung des Organs durch Nahrungs- und Flüssigkeitskarenz, Absaugen des Magensekrets und Hemmung der Pankreassekretion durch Azetazolamid, Calcitonin, Glucagon, Cimetidin, Hymecromon, oder chirurgische Maßnahmen, wie Laparotomie (Abszesseröffnung und Nekrosenausräumung, Choledochusdrainage, partielle oder (sub-)totale Resektion des Pankreas, Sanierung der Gallenwege (Papillotomie) und allgemeine Therapien, wie Antibiotika (wegen der Invasion von Darmbakterien in das entzündete Pankreas), Schocktherapie (wegen des hohen Flüssigkeits- und Plasmaverlustes), Schmerzbekämpfung und Verabreichung von Kortikoiden.

Tenner et al. (World J. Surg. 21 (1997), S. 143-148) konnten jedoch feststellen, dass die "no oral intake" (NPO)-Behandlung (also Nahrungs- und Flüssigkeitskarenz) sowie die Behandlung mit anticholinergen Substanzen und H₂-Blockern weder zu einer Verbesserung der mit der Krankheit verbundenen Schmerzen, noch zur Verkürzung der Länge des Krankenhausaufenthaltes führen konnten. Ebenso konnte nachgewiesen werden, dass die Behandlung mit Somatostatin oder Octreotid, Aprotinin (Trasylol) und anderen Proteaseinhibitoren, sowie Gabexatmesilat, Atropin, Glucagon oder Calcitonin, in klinischen Versuchen keine Wirksamkeit ergaben und daher zur Therapie von Pankreatitis nicht wirklich zu empfehlen sind.

Die Behandlung mit Antibiotika, wie Ciprofloxacin, Ofloxacin und Imipenem führte hingegen zu einer signifikanten Reduktion von pankreatischer Sepsis und zu einer ebenfalls signifikanten Reduktion der Mortalität infolge gram-negativer Sepsis und sekundärer Infektion. Diese Effekte sind allerdings ausschließlich auf die antibakterielle Wirkung der Antibiotika zurückzuführen und nicht auf eine Bekämpfung der eigentlichen Ursachen der Pankreatitis.

Aus Mangel an geeigneten chemisch-medizinischen Behandlungsverfahren stellt daher die Betreuung von Pankreatitispatienten auf der Intensivstation mit Maßnahmen, wie rigoroser Hydration, Elektrolytüberwachung, Sauerstoffzufuhr, frühzeitiger kompletter parenteraler Ernährung, in Verbindung mit chirurgischen Maßnahmen, wie Gallenstein-Entfernung und/oder Absaugen der Nekroseherde mit geleiteter perkutaner Feinnadel-Aspiration (GPA) (jeweils nach Untersuchung mittels endoskopischer retrograder Cholangiopankreatographie), gegebenenfalls in Kombination mit Antibiotika, derzeit die einzig wirksame Therapie für Pankreatitis dar (siehe Tenner et al. (1997)).

Es besteht daher ein großer Bedarf an einer medizinisch-chemischen Behandlung der Pankreatitis, die alleine oder in Kombination mit den intensiv-medizinischen Behandlungsverfahren zur Therapie der Pankreatitis, insbesondere von akuter Pankreatitis, eingesetzt werden kann.

Aufgabe der vorliegenden Erfindung ist daher das zur Verfügung stellen einer medizinisch-chemischen Behandlungsmethode, die alleine oder in Kombination mit bekannten Heilverfahren gegen Pankreatitis eingesetzt werden kann.

Diese Aufgabe wird erfindungsgemäß gelöst durch die Verwendung von Orosomucoid zur Herstellung einer pharmazeutischen Präparation zur Behandlung von akuter Pankreatitis.

Überraschenderweise hat sich gezeigt, dass Orosomucoid in anerkannten Pankreatitis-Tiermodellen, wie sie in Lerch et al. (1994) und Feddersen et al. (Int. J. Pancreatol. 8 (1991), S. 323-331) beschrieben werden, eine verblüffende Wirksamkeit zeigte. Im Tiermodell der ödematösen Pankreatitis konnte eine deutlich verminderte Ödembildung und Vakuolisierung durch Gabe von Orosomucoid erzielt werden.

Es konnte mit der vorliegenden Erfindung im Tiermodell der hämorrhagisch/nekrotischen Pankreatitis gezeigt werden, dass mit der Verabreichung von Orosomucoid den nekrotischen Prozessen, insbesondere der Autolyse, effizient entgegengewirkt werden konnte. Dies stellt eine völlig neuartige medizinische Indikation für Orosomucoid dar.

Das Einsatzgebiet der vorliegenden Erfindung liegt, wie erwähnt, bei der Behandlung von akuter Pankreatitis, insbesondere, wenn eine ödematöse oder nekrotisierende Pankreatitis vorliegt.

Es zeigte sich auch, dass erfindungsgemäß ein durch Pankreatitis hervorgerufener Organschaden an anderen Organen, wie z.B. der Lunge, durch die erfindungsgemäße Orosomucoid-Pankreatitis-Behandlung wirksam bekämpft werden kann. Insbesondere kann damit das bekanntermaßen in Zusammenhang mit Pankreatitis auftretende ARDS (adult respiratory distress syndrome) bekämpft werden. ARDS ist oft eine lebensbedrohliche Komplikation in Pankreatitispatienten, wobei die Ursache und der Zusammenhang von Pankreatitis mit ARDS noch weitgehend unklar ist. Es gibt jedoch ein Tiermodell (Feddersen et al. (1991)), mit welchem diese Zustände untersucht werden können. Erfindungsgemäß kann daher ein durch Pankreatitis hervorgerufener Schaden der Lunge, eventuell der Niere und der Leber ebenfalls effizient behandelt werden.

Auch in Zusammenhang mit einer durch Pankreatitis hervorgerufenen Sepsis wird Orosomucoid erfindungsgemäß zur Verfügung gestellt, wobei auch die Kombination mit Antibiotika vorgeschlagen wird.

Die Herstellungsweise von Orosomucoid ist bekannt (beispielsweise aus der WO 95/07703); als Quelle für humanes Orosomucoid dient vorzugsweise humanes Plasma bzw. eine Plasmafraktion, beispielsweise eine COHN-Fraktion, wie COHN IV oder COHN V. Gemäß einer bevorzugten Ausführungsform der vorliegenden Erfindung wird zur Behandlung von akuter Pankreatitis eine pharmazeutische Präparation mit einem plasmatischen Orosomucoid zur Verfügung gestellt.

Alternativ kann das erfindungsgemäß verwendete Orosomucoid auch rekombinant hergestellt werden (Merritt et al., Gene 66 (1998), Seiten 97-106; wobei bei der rekombinanten Herstellung die Glykosylierung, insbesondere der Sialylierungsgrad von Orosomucoid, variiert werden kann, siehe beispielsweise Williams et al. (1997).

Erfindungsgemäß wird bevorzugterweise ein Orosomucoid mit einem physiologischen Sialylierungsgrad von etwa 11 % verwendet. Dieser Wert wird ermöglicht durch Herstellung des Orosomucoid-Präparates aus gepooltem Humanplasma.

Bevorzugterweise kann die erfindungsgemäße pharmazeutische Orosomucoid-Präparation weiters Albumin enthalten.

Die erfindungsgemäße Pankreatitis-Behandlungspräparation weist als Flüssigpräparat vorzugsweise einen Aluminiumgehalt von weniger als 100 µg/l, vorzugsweise weniger als 10 µg/l, insbesondere weniger als 200 ng/l, auf. Geeignete Verfahren zur Herstellung von Aluminium-reduzierten, lagerstabilen pharmazeutischen Präparationen sind beispielsweise in der WO 98/12225 oder in der EP 0 484 464 zitiert.

Die Applikation der Orosomucoidpräparation kann erfindungsgemäß auf alle Arten erfolgen, insbesondere i.v., s.c., i.m. sowie lokal, wobei die intravenöse Applikation besonders bevorzugt wird. Die angewendete Dosis ist abhängig von der Schwere des Zustandes des Patienten, wobei eine tägliche Einzeldosis von 0,05 bis 1 g/kg Körpergewicht eine bevorzugte Richtgröße darstellt, von der erfindungsgemäß bevorzugt ausgegangen wird.

Vorzugsweise wird der pharmazeutischen Präparation vor der erfindungsgemäßen Verwendung ein Stabilisator zugemischt, insbesondere - neben Albumin - Natriumcaprylat und gegebenenfalls Tenside, um die Lagerstabilität bzw. die Stabilität während einer Hitzebehandlung zu erhöhen.

Es ist auch zweckmäßig, die pharmazeutische Präparation zur Inaktivierung bzw. Abreicherung von Viren zu behandeln, insbesondere durch mindestens eine physikalische Behandlung, wie Hitzebehandlung und/oder Filtration. Zur Inaktivierung von Viren sind eine Reihe von physikalischen, chemischen oder chemisch-physikalischen Methoden bekannt, wie beispielsweise eine Hitzebehandlung, z.B. eine Pasteurisierung oder gemäß der EP 0 159 311 A oder der EP 0 637 451 A, eine Hydrolysebehandlung gemäß der EP 0 247 998 A oder eine Strahlenbehandlung oder eine Behandlung mit organischem Lösungsmittel und/oder Tensiden, z.B. gemäß der EP 0 131 740 A. Weitere geeignete Virusinaktivierungsschritte bei der Herstellung der erfindungsgemäßen Präparate sind in der EP 0 506 651 A oder in der WO 94/13329A beschrieben.

Die Erfindung wird anhand der nachfolgenden Beispiele und der Zeichnungsfiguren, auf die sie jedoch nicht eingeschränkt sein soll, näher beschrieben.

Es zeigen:
Fig. 1: die Wirkung von Orosomucoid im GDOC-Pankreatitis-Tiermodell, und
Fig. 2: die Wirkung von Orosomucoid im Ratten-Caerulein-Pankreatitis-Modell.

### Beispiele:

### Beispiel 1 : Hämorrhagisch-nekrotische Pankreatitis an Ratten

Nüchternen männlichen Wistarraten (> 220 g) wird in Ketamin/Xylazinnarkose (i.m.) nach Laparotomie der Ductus pancreaticus kanüliert; der Ductus choledochus wird abgeklemmt. In den Ductus pancreaticus erfolgt die retrograde Applikation von Glycodeoxycholsäure (GDOC; 4 mg/0,5 ml/kg über 2 min, danach wird der Ductus mit 0,2 ml isotoner Kochsalzlösung freigespült). Zwei Minuten nach Applikation wird der Ductus pancreaticus-Katheter wieder entfernt, und die Klemme des Ductus choledochus eröffnet. Die Laparotomiewunde wird anschließend wieder verschlossen. Über eine V. jugularis wird ein Dauerkatheter implantiert, der eine Dauerinfusion am frei beweglichen, wachen Tier ermöglicht.

### Behandlungsregime mit Orosomucoid:

**1. Serie:** (24 h Substanzapplikation, danach Tötung der Tiere)
   Behandlungsgruppe: GDOC in D. pancreaticus; gleich danach Bolus von 600 mg/10,75 ml/kg + Infusion von 600 mg/kg/h Orosomucoid über 24 h (i.v.)
   negative Kontrollgruppe: 0,5 ml/kg isotone Kochsalzlösung in den D. pancreaticus; Bolus von 10,75 ml/kg + Infusion von 10,75 ml/kg/h NaCl über 24 h (i.v.)
   positive Kontrollgruppe: GDOC in D. pancreaticus; Bolus von 10,75 ml/kg + Infusion von 10,75 ml/kg/h NaCl über 24 h (i.v.)
   Plazebogruppe: GDOC in D. pancreaticus; Bolus von 10,75 ml/kg + Infusion von 10,75 ml/kg/h Plazeboformulierung über 24 h (i.v.)
**2**. **Serie:** (6 h Substanzapplikation, danach Tötung der Tiere)
   Behandlungsgruppe: GDOC in D. pancreaticus; gleich danach Bolus von 600 mg/10,75 ml/kg + Infusion von 600 mg/kg/h Orosomucoid über 6 h (i.v.)
   negative Kontrollgruppe: 0,5 ml/kg isotone Kochsalzlösung in den D. pancreaticus; Bolus von 10,75 ml/kg + Infusion von 10,75 ml/kg/h NaCl über 6 h (i.v.)
   positive Kontrollgruppe: GDOC in D. pancreaticus; Bolus von 10,75 ml/kg + Infusion von 10,75 ml/kg/h NaCl über 6 h (i.v.)
   Plazebogruppe: GDOC in D. pancreaticus; Bolus von 10,75 ml/kg + Infusion von 10,75 ml/kg/h Plazeboformulierung über 6 h (i.v.)
**3**. **Serie:** (6 h Substanzapplikation, nach 24 h Tötung der Tiere)
   Behandlungsgruppe: GDOC in D. pancreaticus; gleich danach Bolus von 600 mg/10,75 ml/kg + Infusion von 200 mg/10,75 ml/kg/h Orosomucoid über 6 h (i.v.)
   negative Kontrollgruppe: 0,5 ml/kg isotone Kochsalzlösung in den D. pancreaticus; Bolus von 10,75 ml/kg + Infusion von 10,75 ml/kg/h NaCl über 6 h (i.v.)
   positive Kontrollgruppe: GDOC in D. pancreaticus; Bolus von 10,75 ml/kg + Infusion von 10,75 ml/kg/h NaCl über 6 h (i.v.)
   Plazebogruppe: GDOC in D. pancreaticus; Bolus von 10,75 ml/kg + Infusion von Plazeboformulierung im Volumen von 10,75 ml/kg/h über 6 h (i.v.).

Nach Versuchsende werden die Tiere erneut narkotisiert und durch Herzpunktion entblutet. Nach Eröffnen der Bauchhöhle wird Aszitesflüssigkeit gewonnen und deren Volumen bestimmt, sowie Pankreasgewebe zur histologischen Untersuchung entnommen. Aus dem Plasma werden photometrisch Amylase, Lipasen und Laktatdehydrogenase bestimmt.

### Ergebnisse:

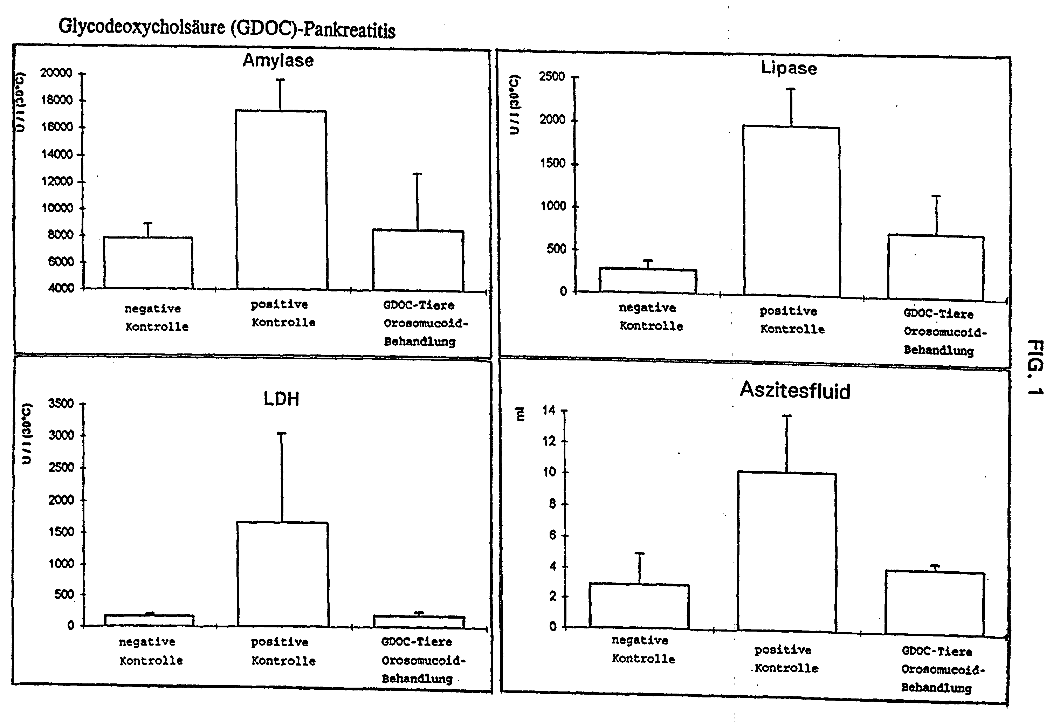

Orosomucoid zeigte in allen Behandlungsgruppen positive Effekte; in Fig. 1 wird ein Überblick über die Ergebnisse der negativen und positiven Kontrollgruppen sowie der Behandlungsgruppen gegeben. Ergebnisse der Plazebogruppen werden im Text besprochen.

### 1. Bauchhöhlenpunktat:

Tiere der positiven Kontrollgruppe bilden eine deutlich größere Menge an Aszitesflüssigkeit im Vergleich zur negativen Kontrollgruppe. GDOC-Tiere, die mit Orosomucoid behandelt wurden, zeigen geringere Mengen als die positive Kontrollgruppe.

### 2. Plasmaparameter:

2.1 Amylase: Tiere der negativen Kontrollgruppe haben Plasmaamylasewerte im Bereich von 8000 E/l. Bei Tieren, die mit GDOC + isotoner Kochsalzlösung behandelt wurden (positive Kontrollgruppe) steigen die Amylasewerte innerhalb von 24 h auf das 2- bis 3fache an. Behandlung mit Orosomucoid kann den Anstieg der Amylase deutlich verringern, die Plazeboformulierung hat keinen positiven Effekt.
2.2 Plasmalipasen: Positive Kontrolltiere haben mehr als doppelt so hohe Lipasewerte wie Tiere der negativen Kontrollgruppe. GDOC-Tiere, denen Orosomucoid infundiert wurde, zeigen niedrigere Lipasewerte. Mit Plazeboformulierung behandelte GDOC-Tiere zeigen zum Teil höhere Lipasewerte als die positive Kontrollgruppe.
2.3 Plasmalaktatdehydrogenase (LDH) : Tiere der negativen Kontrollgruppe haben LDH-Werte im Bereich von etwa 300 E/l. In der positiven Kontrollgruppe sind die Werte deutlich höher. GDOC-Tiere, die mit Orosomucoid behandelt wurden, haben niedrigere LDH-Werte als die Tiere der positiven Kontrollgruppe. Bei GDOC-Tieren, denen Plazeboformulierung infundiert wurde, steigen die LDH-Werte deutlich an.

### 3. Histologie:

Bei der histologischen Untersuchung von Pankreasgewebe zeigten die Tiere der positiven Kontrollgruppe deutliche Anzeichen einer aktiven hämorrhagisch-nekrotischen Pankreatitis. So konnten massive Nekrosen der Azinarzellen, Ödeme, Blutungen sowie deutliche Infiltration mit Entzündungszellen beobachtet werden. Tiere, die isotone Kochsalzlösung in den D. pancreaticus + isotone Kochsalzlösung intravenös verabreicht bekamen (negative Kontrollgruppe), zeigten vereinzelt ödemisierung sowie geringgradige Blutungen im Pankreasgewebe, niemals jedoch Nekrosen. Behandlung von GDOC-Tieren mit Orosomucoid (Behandlungsgruppe) konnte die Ausbildung einer hämorrhagisch-nekrotischen Pankreatitis deutlich mildern. Bei GDOC-Tieren, die Plazeboformulierung infundiert erhielten (Plazebogruppe), waren dagegen massiv nekrotische Pankreasazinarzellen aufzufinden.

### Beispiel 2 :

An wachen Ratten wurde durch Infusion von 10 µg/kg h⁻¹ Caerulein i.v. über 6 h eine ödematöse Pankreatitis erzeugt. Als Parameter wurden der Anstieg von Amylase im Plasma und der Wassergehalt des Pankreas untersucht sowie eine histologische Untersuchung durchgeführt.

In den vorliegenden Untersuchungen wurde Orosomucoid mit 600 mg/kg i.v. als Bolus vor der Caeruleininfusion appliziert sowie mit 200 mg/kg h⁻¹ parallel zur Caeruleininfusion.

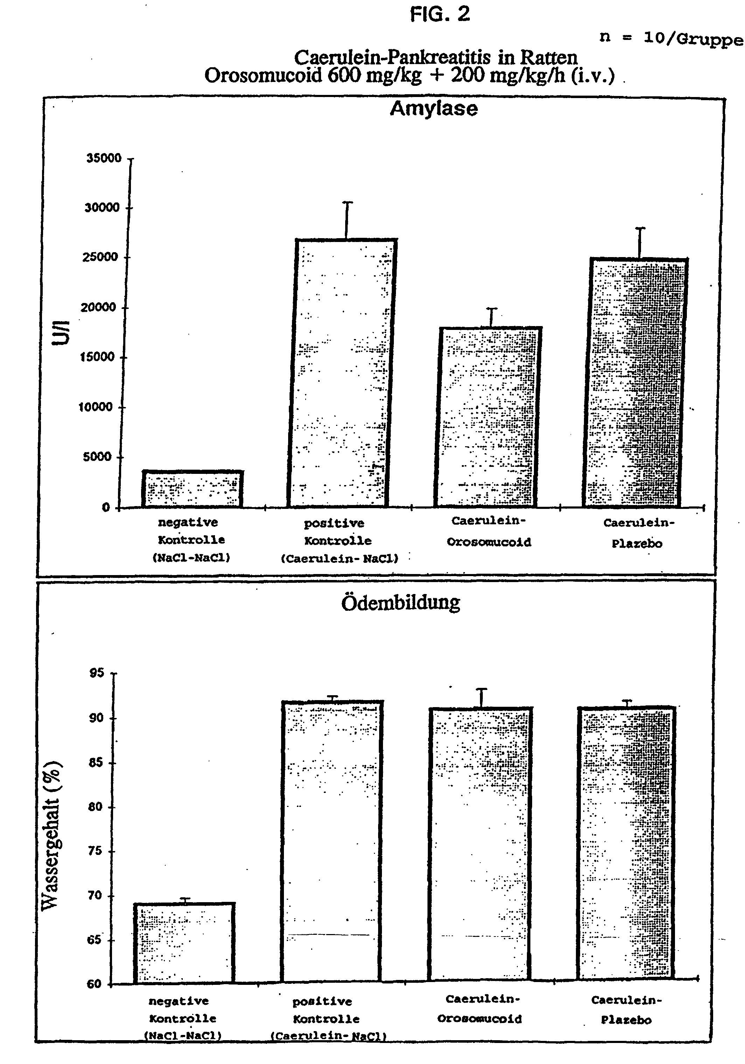

Die Ergebnisse sind in Fig. 2 dargestellt. Caerulein steigerte die Plasmaamylaseaktivität dramatisch (von 3600 E/l an negativen Kontrollen auf 26700 E/l). Eine Gabe von Orosomucoid senkte diesen Wert signifikant auf 18000 E/l. Die Plazeboformulierung blieb ohne diesen Effekt (24800 E/l). Interessanterweise blieb Orosomucoid in diesem Modell ohne Wirkung auf das Pankreasödem. Der Wassergehalt stieg von 69 % in negativen Kontrollen auf 91,6 % in Caerulein-behandelten Tieren und dieser Effekt wurde weder durch Orosomucoid (90,6 %) noch durch die Plazeboformulierung (90,6 %) beeinflusst.

Bei dieser histologischen Untersuchung von Pankreasgewebe zeigten Tiere der positiven Kontrollgruppe (Caeruleininfusion + isotone Kochsalzlösung) deutliche Anzeichen einer akuten ödematösen Pankreatitis. So konnte die massive Ausbildung eines interstitiellen Ödems sowie hochgradige Vakuolisierung der Azinarzellen beobachtet werden.

Tiere, die nur isotone Kochsalzlösung infundiert bekamen (negative Kontrollgruppe), zeigten in keinem Fall pathologisch verändertes Pankreasgewebe.

Caerulein-vorbehandelte Tiere, denen zusätzlich Orosomucoid verabreicht worden war (Behandlungsgruppe), zeigten im histologischen Bild eine verminderte ödembildung.

Die durch Caerulein bedingte Vakuolisierung der Zellen des exokrinen Pankreas wird durch Orosomucoid-Behandlung deutlich vermindert.

Bei Tieren, die zusätzlich zur Caeruleininfusion Plazeboformulierung erhalten hatten, waren wiederum massive Ödembildung sowie äußerst zahlreiche Vakuolen in den Pankreasazinarzellen aufzufinden.

Damit konnte erstmals eine günstige Beeinflussung einer experimentellen Pankreatitis durch Orosomucoid gezeigt werden, was einen völlig neuen Befund darstellt.

## Patentansprüche

1. Verwendung von Orosomucoid zur Herstellung einer pharmazeutischen Präparation zur Behandlung von akuter Pankreatitis.

2. Verwendung nach Anspruch 1, **dadurch gekennzeichnet, dass** die Pankreatitis eine ödematöse Pankreatitis ist.

3. Verwendung nach Anspruch 1, **dadurch gekennzeichnet, dass** die Pankreatitis eine nekrotisierende Pankreatitis ist.

4. Verwendung nach einem der Ansprüche 1 bis 3, **dadurch gekennzeichnet, dass** die Präparation zur Behandlung eines durch die Pankreatitis hervorgerufenen Organschadens bestimmt ist.

5. Verwendung nach Anspruch 4, **dadurch gekennzeichnet, dass** die Präparation zur Behandlung eines durch die Pankreatitis hervorgerufenen Schadens der Lunge bestimmt ist.

6. Verwendung nach einem der Ansprüche 1 bis 5, **dadurch gekennzeichnet, dass** die Präparation zur Behandlung einer durch die Pankreatitis hervorgerufenen Sepsis bestimmt ist.

7. Verwendung nach einem der Ansprüche 1 bis 6, **dadurch gekennzeichnet, dass** die pharmazeutische Präparation ein plasmatisches Orosomucoid enthält.

8. Verwendung nach einem der Ansprüche 1 bis 6, **dadurch gekennzeichnet, dass** die pharmazeutische Präparation ein von einer rekombinanten Nukleinsäure kodiertes Orosomucoid enthält.

9. Verwendung nach Anspruch 7 oder 8, **dadurch gekennzeichnet, dass** das Orosomucoid einen physiologischen Sialylierungsgrad aufweist.

10. Verwendung nach einem der Ansprüche 1 bis 9, **dadurch gekennzeichnet, dass** die pharmazeutische Präparation weiters Albumin enthält.

11. Verwendung nach einem der Ansprüche 1 bis 10, **dadurch gekennzeichnet, dass** die pharmazeutische Präparation durch einen Aluminiumgehalt von weniger als 100 µg/l gekennzeichnet ist.

12. Verwendung nach einem der Ansprüche 1 bis 11, **dadurch gekennzeichnet, dass** die pharmazeutische Präparation zur intravenösen Verabreichtung bestimmt ist.

13. Verwendung nach Anspruch 12, **dadurch gekennzeichnet, dass** die Dosis 0,05 - 1 g/kg tägliche Einzeldosis beträgt.

## Claims

1. The use of Orosomucoid for producing a pharmaceutical preparation for the treatment of acute pancreatitis.

2. The use according to claim 1, **characterized in that** the pancreatitis is an edematous pancreatitis.

3. The use according to claim 1, **characterized in that** the pancreatitis is a necrotizing pancreatitis.

4. The use according to any one of claims 1 to 3, **characterized in that** the preparation is intended for the treatment of an organ injury caused by pancreatitis.

5. The use according to claim 4, **characterized in that** the preparation is intended for the treatment of an injury of the lungs caused by pancreatitis.

6. The use according to any one of claims 1 to 5, **characterized in that** the preparation is intended for the treatment of a sepsis caused by pancreatitis.

7. The use according to any one of claims 1 to 6, **characterized in that** the pharmaceutical preparation comprises a plasmatic Orosomucoid.

8. The use according to any one of claims 1 to 6, **characterized in that** the pharmaceutical preparation comprises an Orosomucoid encoded by a recombinant nucleic acid.

9. The use according to claim 7 or 8, **characterized in that** the Orosomucoid has a physiological sialylation level.

10. The use according to any one of claims 1 to 9, **characterized in that** the pharmaceutical preparation further comprises albumin.

11. The use according to any one of claims 1 to 10, **characterized in that** the pharmaceutical preparation is **characterized by** an aluminum content of less than 100 µg/l.

12. The use according to any one of claims 1 to 11, **characterized in that** the pharmaceutical preparation is intended for intravenous administration.

13. The use according to claim 12, **characterized in that** the dose is 0.05 - 1 g/kg as a daily single dose.

## Revendications

1. Utilisation d'orosomucoïde pour fabriquer une préparation pharmaceutique destinée au traitement de la pancréatite aiguë.

2. Utilisation selon la revendication 1, **caractérisée en ce que** la pancréatite est une pancréatite oedémateuse.

3. Utilisation selon la revendication 1, **caractérisée en ce que** la pancréatite est une pancréatite nécrosante.

4. Utilisation selon l'une des revendications 1 à 3, **caractérisée en ce que** la préparation est destinée au traitement d'une lésion organique provoquée par la pancréatite.

5. Utilisation selon la revendication 4, **caractérisée en ce que** la préparation est destinée au traitement d'une lésion pulmonaire provoquée par la pancréatite.

6. Utilisation selon l'une des revendications 1 à 5, **caractérisée en ce que** la préparation est destinée au traitement d'une septicémie provoquée par la pancréatite.

7. Utilisation selon l'une des revendications 1 à 6, **caractérisée en ce que** la préparation pharmaceutique contient un orosomucoïde plasmatique.

8. Utilisation selon l'une des revendications 1 à 6, **caractérisée en ce que** la préparation pharmaceutique contient un orosomucoïde codé par un acide nucléique recombinant.

9. Utilisation selon la revendication 7 ou la revendication 8, **caractérisée en ce que** l'orosomucoïde présente un degré de silylation physiologique.

10. Utilisation selon l'une des revendications 1 à 9, **caractérisée en ce que** la préparation pharmaceutique contient en plus de l'albumine.

11. Utilisation selon l'une des revendications 1 à 10, **caractérisée en ce que** la préparation pharmaceutique est **caractérisée par** une teneur en aluminium inférieure à 100 µg/l.

12. Utilisation selon l'une des revendications 1 à 11, **caractérisée en ce que** la préparation pharmaceutique est destinée à l'administration par voie intraveineuse.

13. Utilisation selon la revendication 12, **caractérisée en ce que** la dose est une dose journalière unique de 0,05 à 1 g/kg.
